# EUROPEAN PATENT APPLICATION

(11) **EP 2 772 447 A1**
(43) Date of publication of application: **03.09.2014**
(21) Application number: 12842987.5
(22) Date of filing: 23.10.2012
(51) Int. Cl.: B65B 55/10, B65B 55/04, B65B 55/06, B65B 55/24

(54) **BEVERAGE FILLING METHOD AND APPARATUS**

(30) Priority: 25.10.2011 JP 2011233680
(71) Applicant: Dai Nippon Printing Co., Ltd., Tokyo 162-8001 (JP)
(72) Inventor: HAYAKAWA Atsushi, Tokyo 162-8001 (JP); HIDAKA Hideo, Tokyo 162-8001 (JP)
(74) Representative: Viering, Jentschura & Partner Patent- und Rechtsanwälte
(86) International application number: PCT/JP2012/077336
(87) International publication number: WO 2013/061956

(57) **Abstract**

A drink package is manufactured by a method including the steps of: blasting hydrogen peroxide mist (M) or gas (G) into a container (2) in an erected attitude through a mouth portion (2a); activating the hydrogen peroxide in the container (2) in an inverted attitude by blasting heated aseptic air (N) through the mouth portion (2a) thereof directed downward, and simultaneously, removing foreign substance (P) from the inside of the container (2) by replacing air in the container (2) with the aseptic air (N); filling the container (2) in the erected attitude with drink (a) through the mouth portion (2a) directed upward; and closing the mouth portion (2a) of the container (2) by applying a lid (3) to the mouth portion (2a) of the container (2).

## Description

### Technical Field

The present invention relates to a drink filling method and a drink filling system.

### Background Technology

Conventionally, there is provided an aseptic filling method as an inline system (for example, refer to Patent Documents 1 and 2) in which, while conveying preforms, a sterilizing agent such as hydrogen peroxide is blasted to the preform, the preform is then heated to thereby activate the sterilizing agent adhering to a surface of the preform and increase a temperature of the preform to a temperature suitable for molding the preform, thereafter, the preform is deformed into a bottle by a blow-molding machine, and the bottle is then filled up with drink and capped as an aseptic package.

### Prior Art Document

### Patent Document

Patent Document 1: Japanese Translation of PCT international Application Publication No. 2008-546605
Patent Document 2: Japanese Patent Publication No. 3903411
Patent Document 3: Japanese Patent Publication No. 4012653

### Summary of The Invention

### Problems to be solved by The Invention

The conventional technology mentioned above is a method in which, in substitution for the sterilization treatment after the bottle molding process, a preform before the bottle molding process is sterilized and a drink fills the bottle immediately after the molding of the bottle, and hence, it is necessary to supply much amount of the sterilizing agent so as not to cause insufficient sterilization. However, if much amount of the sterilizing agent adheres to the surface of the preform, there is a fear that the bottle may be discoloured and/or deformed at the bottle molding process. In addition, in a case when less amount of the sterilizing agent is supplied to prevent such defect, there is a fear that bacteria or like remains alive.

Furthermore, there may cause a case wherein foreign substance or material such as fine plastic chip or piece, or like is confused into a bottle at the time of blow molding to form the bottle by using highly pressurized air without being limited to the inline system mentioned above. Moreover, there may also cause a case wherein a sterilizing agent such as hydrogen peroxide or like remains in the bottle. In a conventional technology, in order to remove such foreign material and remaining sterilizing agent from the bottle, a rinsing is performed by supplying aseptic water into the bottle before filling of a content (for example, refer to Patent Document 3).

However, in the case where the bottle is cleaned by using the aseptic water, a drink filling system is itself enlarged, and a large amount of energy for manufacturing the aseptic water is consumed, thus providing a problem.

An object of the present invention is to solve the above-mentioned problem.

### Means for solving The Problem

To achieve the above object, the present invention adopts the following configuration.

It is further to be noted that, in the following, although reference numerals described in the drawings are added with parentheses, the present invention is not limited to them.

That is, the invention according to claim 1 adopts a drink filling method including the steps of: blasting hydrogen peroxide mist (M) or gas (G) into a container (2) in an erected attitude through a mouth portion (2a); activating the hydrogen peroxide in the container (2) in an inverted attitude by blasting heated aseptic air (N) through the mouth portion (2a) thereof directed downward, and simultaneously, removing foreign substance (P) from the inside of the container (2) by replacing air in the container (2) with the aseptic air (N); filling the container (2) in the erected attitude with drink (a) through the mouth portion (2a) directed upward; and closing the mouth portion (2a) of the container (2) by applying a lid (3) to the mouth portion of the container.

As described in claim 2, in the drink filling method recited in claim 1, it may be desired that, while continuously traveling the preforms (1), each preform (1) is heated to a temperature suitable for molding, the preform (1) is blow-molded into the container (2), and steps from the hydrogen peroxide mist (M) or gas (G) blasting step to the container mouth portion closing step with the lid (3) are subsequently performed with respect to the container (2) having remaining heat at the time of above-mentioned heating step.

As described in claim 3, in the drink filling method recited in claim 2, it may be desired that while continuously traveling the preforms (1), the hydrogen peroxide mist (K) or gas is contacted to the preforms (1) and steps form the preform heating step in which the preform (1) is heated to the molding temperature to the container mouth portion closing step for closing the lid (3) thereof are subsequently performed.

As described in claim 4, in the drink filling method recited in claim 1, it may be desired that the aseptic air is blasted into the container by a flow rate of 100L/min. to 500L/min. for 3 to 20 sec.

Further, the above flow rate is preferably of 150L/min. to 300L/min.

As described in claim 5, in the drink filling method recited in claim 2 or 3, it may be desired that air (Q) is blasted into the preform (1) before heating the preform to the molding temperature to thereby remove foreign substance (P) from the preform (1).

The invention described in claim 6 adopts a drink filling system, in which there is provided a conveying path for continuously travelling a preform (1) and a container (2) during processes in which a preform (1) is formed to a container (2), a drink (a) fills the container (2), the container (2) is sealed by a lid (3), and for traveling the container (2) in a temporarily inverted attitude, the drink filling system including: a heating furnace (50) for heating the preform (1) to a temperature suitable for molding; a mold (4) for blow-molding the heated preform (1) into the mold (4); a sterilizing nozzle (6) for blasting the hydrogen peroxide mist (M) or gas (G) into the blow-molded container (2) through the mouth portion (2a) thereof; an air-rinsing nozzle (45) for activating the hydrogen peroxide inside the container (2) by blasting the heated aseptic air (N) through the mouth portion (2a) of the container (2) directed downward traveling in the inverted attitude and substantially reducing the air inside the container with the aseptic air (N) to thereby remove the foreign substance (P) from the container (2); a drink filling nozzle (39) for filling the container (2) with the drink (a); and a capper (40) for closing the mouth portion (2a) of the container (2) which is filled up with the drink (a), which are arranged along the conveying path.

As described in claim 7, in the drink recited in claim 6, it may be desired that an air nozzle (53) for filling system removing the foreign substance (P) from the inside of the preform (1) by blasting air (Q) into the preform (1) before heating the preform (1) to the molding temperature is provided for the conveying path.

As described in claim 8, in the drink filling system recited in claim 7, it may be desired that a suction nozzle (54) for sucking the foreign substance (P) discharged from the inside of the preform (1) is provided for the conveying path.

### Effects of The Invention

According to the invention recited in claims 1 to 6, the interior of the container (2) can be sterilized by blasting the hydrogen peroxide mist (M) or gas (G) into the container (2) through the mouth portion (2a) thereof, as well as the enhancement of the sterilized effect by activating the hydrogen peroxide inside the container (2) by the heated aseptic air (N), and the remaining amount of the hydrogen peroxide in the container can be reduced. In addition, by replacing the air inside the container (2) with the aseptic air (N), the foreign substance (P) can be discharged through the mouth portion (2a) of the container (2) directed downward and falls downward, so that the foreign substance (P) can be surely rejected outside the container (2). Accordingly, the packaged drink having high quality can be produced.

As described in claim 2, in the drink filling method recited in claim 1, while continuously traveling the preforms (1), each of the preforms (1) is heated to a temperature suitable for molding, the preform (1) is blow-molded into the container (2), and steps from the hydrogen peroxide mist (M) or gas (G) blasting step to the container mouth portion closing step with the lid (3) are subsequently performed with respect to the container (2) having remaining heat at the time of above-mentioned heating step. Accordingly, in a combined function of the remaining heat of the container (2) and the blasting of the heated aseptic air (N), the sterilizing effect by the hydrogen peroxide can be enhanced.

Further, as described in claim 3, in the drink filling method recited in claim 2, while continuously traveling the preforms (1), the hydrogen peroxide mist (K) or gas is contacted to the preforms (1) and steps form the preform heating step in which the preform (1) is heated to the molding temperature to the container mouth portion closing step with the lid (3) thereof are subsequently performed. Accordingly, the use amount of the hydrogen peroxide at the time of the sterilization of the container (2) can be reduced, and in association with the blasting of the heated aseptic air (N), the remaining amount of the hydrogen peroxide in the container can be reduced.

As described in claim 5 or 7, in the case when the air (Q) is blasted into the preform (1) before heating the preform to the molding temperature to thereby remove foreign substance (P) from the preform (1), the foreign substance (P) can be more properly discharged.

### Brief Description of the Drawings

[Fig. 1] is an illustration for explaining respective steps A, B and C of a filling method according to a first embodiment of the present invention.
[Fig. 2] is an illustration for explaining respective steps D and E of the filling method according to the first embodiment of the present invention.
[Fig. 3] is an illustration for explaining respective steps G1 or G2, H and I of the filling method according to the first embodiment of the present invention.
[Fig. 4] is a schematic plan view showing a configuration of one example of a filling system according to the first embodiment of the present invention.
[Fig. 5] is a vertical sectional view showing a device for supplying hydrogen peroxide mist or gas to a preform.
[Fig. 6] is a vertical sectional view showing one example of a mist generator for producing the hydrogen peroxide mist or gas.
[Fig. 7] is a graph representing a foreign material removing rate by performing an air rinsing process, in which (A) shows a case in which air flow rate is set to 150L/min. and 200L/min. with respect to a bottle having a volume of 2L, and (B) shows a case in which air flow rate is set to 200L/min. with respect to a bottle having a volume of 500mL.
[Fig. 8] illustrates one process of a filling method according to a second embodiment of the present invention, in which (A) is a view explaining a step performed before the process of Fig. 1(A) of the first embodiment, and (B) is a view explaining a modified example thereof.

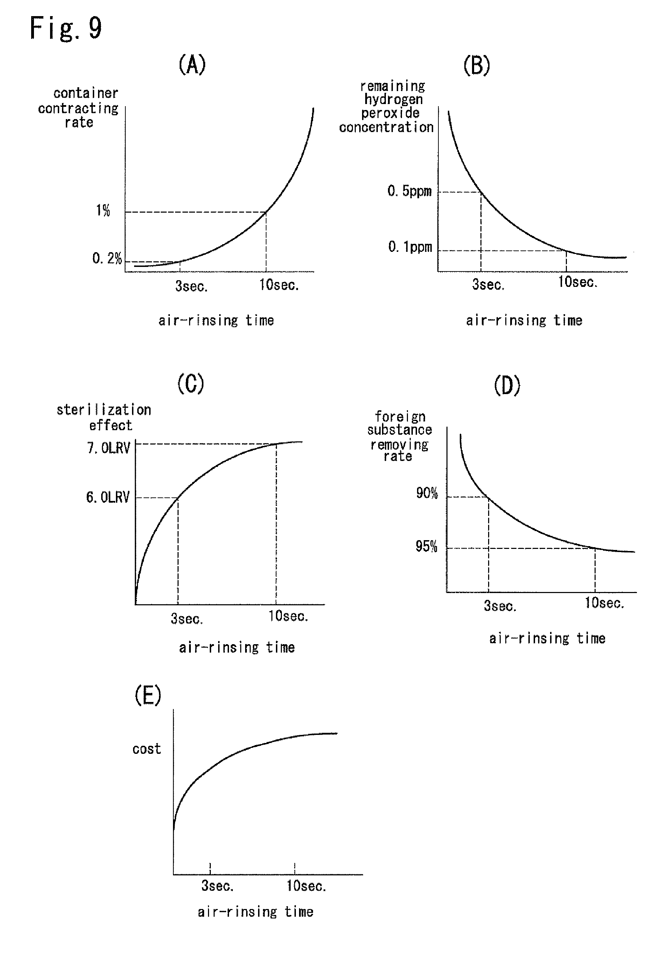

[Fig. 9] shows examples according to the second embodiments of the present invention, in which (A) is a graph representing a relationship between contractile property of a container and an air rinsing time, (B) is a graph representing a relationship between a remaining hydrogen peroxide and an air rinsing time, (C) is a graph representing a relationship between sterilizing effect and an air rinsing time, (D) is a graph representing a relationship between foreign substance or material removing performance and an air rinsing time, and (E) is a graph representing a relationship between cost and an air rinsing time.

### Embodiment for carrying out The Invention

Hereunder, embodiments for carrying out the present invention will be explained.

### <Embodiment 1>

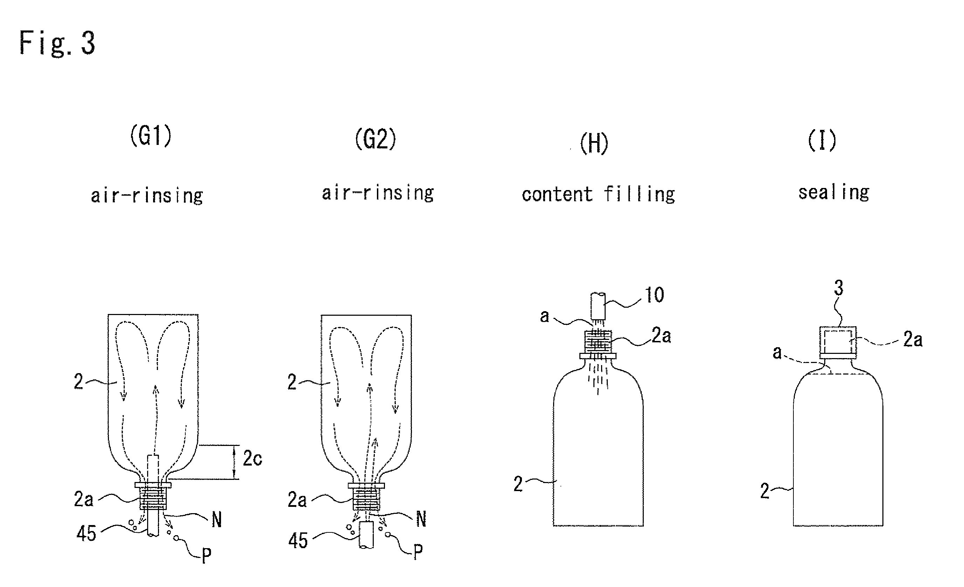

According to an inline system of the first embodiment 1, a package shown in Fig. 3(I) can be manufactured as a final product.

This package is composed of a bottle 2 as a container and a cap 3 as a lid.

In this embodiment, although the bottle 2 is made of PET, another resin such as polypropylene, polyethylene or like may be used for manufacturing the bottle without limiting to the PET, and a male screw (threaded) portion is formed to a mouth portion 2a of the bottle 2.

The cap 3 is produced by an injection molding process with polypropylene being used as a resin material, and a female screw (threaded) portion is also formed together with the molding of the cap 3.

The bottle 2, the interior of which is preliminarily sterilized, is filled up with drink (beverage) "a" which had already been sterilized, and after the filling of the drink "a", the cap 3 is applied to the mouth portion 2a of the bottle 2, which is then sealed by the screw-engagement between the male and female screws, thus completing the package.

The bottle 2 mentioned above is formed according to the following processes, which will be described hereinafter, as a container, and the container is then filled up with the drink and sealed as the package.

First, the preform 1 is continuously conveyed or travelled at a constant speed.

The preform 1 is formed as a bottomed cylindrical body having substantially test tube shape through the PET injection molding process. The preform 1 is formed with a mouth portion 2a as like as that for the bottle 2 shown in Fig. 3(I) at a time of an initial molding process. This mouth portion 2a is formed with the male screw 2b at the same time of molding the preform 1.

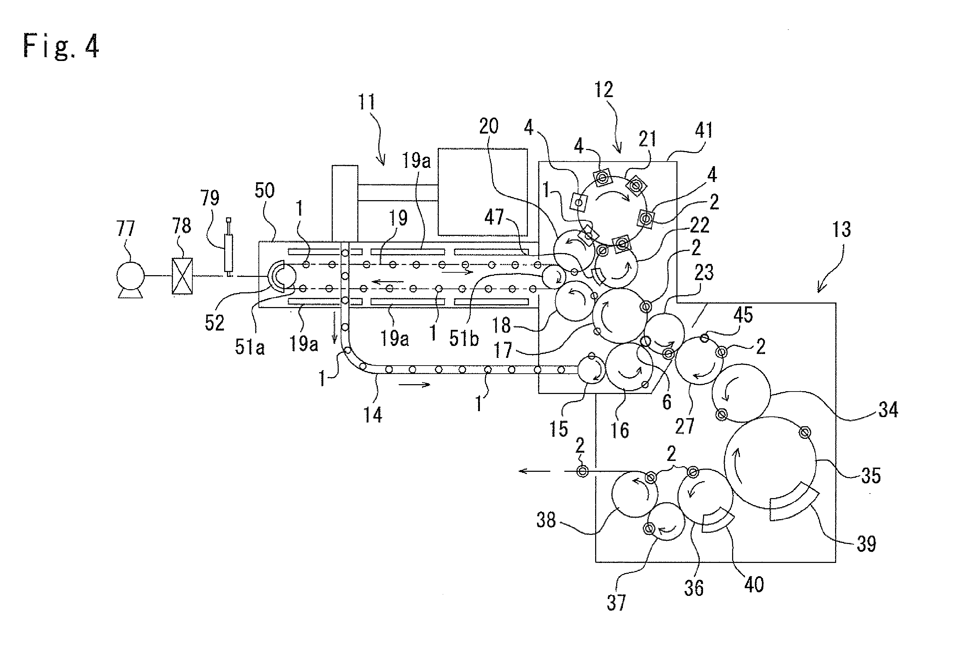

Just after the starting of the conveyance of the preform 1, the preform is preliminary heated. More specifically, the preform 1 is conveyed into a heating furnace 50 shown in Fig. 4 to be thereby preheated.

The heating furnace 50 has a furnace room elongated in one direction. Inside the furnace room, an endless chain 19 is stretched between a pair of pulleys 51a and 51 b disposed in face-to-face manner on a horizontal plane. The endless chain 19 and other associated members constitutes an endless conveyer for conveying a number of preforms 1 in a suspended fashion. Infrared heaters 19a are attached to an inner wall surface of the furnace room along outward path and return path of the endless chain 19.

When the preform 1 is conveyed inside the heating furnace 50, the preform 1 is heated by the infrared heaters 19a while travelling on the outward path of the endless chain 19. During this traveling on the outward path, the preform 1 is preheated to a temperature of about 40 to 80 °C.

However, the heating temperature to the mouth portion 2a of the preform 1 is suppressed to a temperature such as less than 50 °C so as not to deform the preform 1.

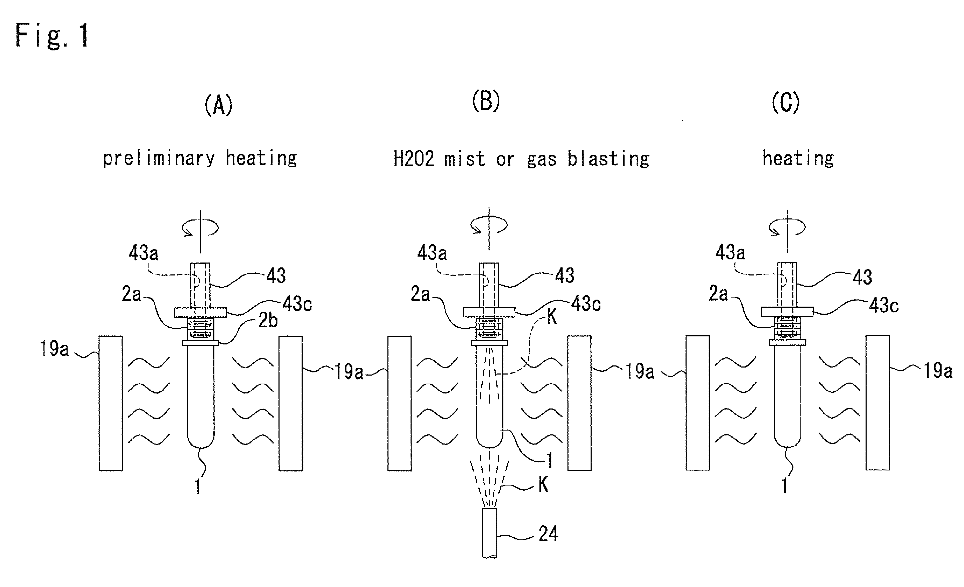

Further, as shown in Fig. 1(A), a spindle 43 is inserted into the mouth portion 2a of the preform 1 in an upright suspended fashion, and under this state, the preform 1 is travelled while being rotated together with the spindle 43. According to this way, the preform 1 can be uniformly preheated by the infrared heaters 19.

Further, a mandrel may be inserted in place of the spindle 43, and in this case, the preform 1 will be travelled, while being rotated, in an inverted fashion together with the mandrel.

Next, as shown in Fig. 1(B), hydrogen peroxide mist K or gas is supplied into the preform 1 now travelling to thereby perform a preliminary sterilization. The supply of the hydrogen peroxide mist K or gas is performed at a time of completion of the preheating of the preform 1. For example, the supply is performed at a time at which the preform 1 is moved from the outward path to the return path of the endless chain 19.

The supply of the hydrogen peroxide mist K or gas is specifically performed by forming a though hole 43a on an axial portion of the spindle 43 and blasting the hydrogen peroxide into the through hole by using the spindle 43 as a nozzle for blasting the hydrogen peroxide.

The hydrogen peroxide mist K or gas to be blasted into the preform 1 is generated by a mist generator of a structure similar to that of a mist generator 7 described hereinafter. The hydrogen peroxide mist K or gas flows inside the preform 1 through the spindle 43, and is in contact with the inner peripheral surface of the preform 1 to thereby form a condensed film of the hydrogen peroxide of 35 weight% conversion, and the hydrogen peroxide mist or gas adheres to the inner peripheral surface in a range preferably of 0.001 µL/cm² to 0.1 *µ*L/cm².

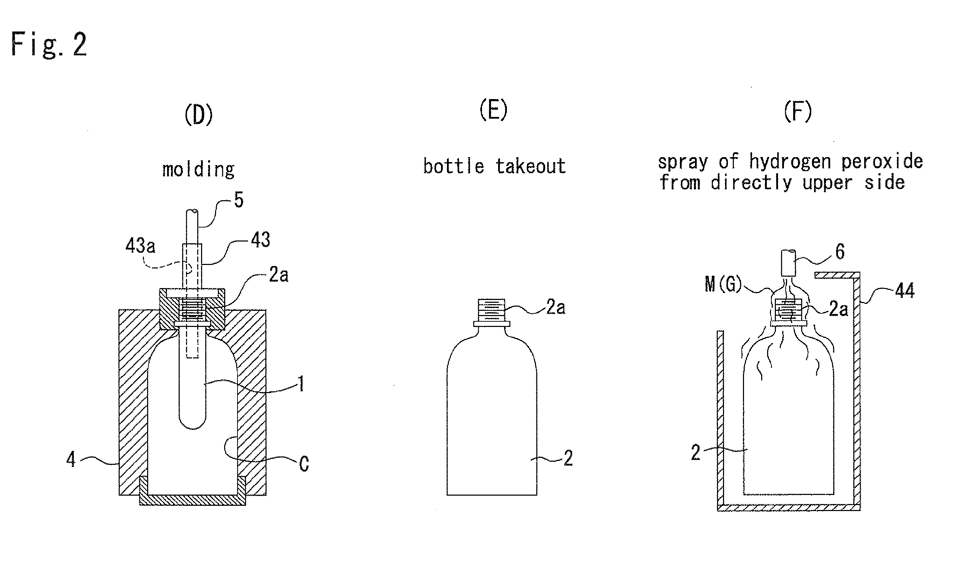

In a case of adhering amount of less than 0.001 *µ* L/cm², sufficient sterilization as preliminary sterilization cannot be obtained. On the other hand, in a case of adhering amount of more than 0.1 *µ* L/cm², the bottle 2 may cause a molding defect such as whitening, spot, winkling, or deformation at the blow molding process as shown in Fig. 2(D).

The adhering amount of the hydrogen peroxide condensed film of 35 weight% conversion to the preform 1 is more preferably in a range of 0.002 *µ* L/cm² to 0.008 *µ* L/cm².

As mentioned above, since the hydrogen peroxide condensed film adheres to the preheated preform 1, the hydrogen peroxide is activated on the surface of the preform 1 to thereby improve the sterilizing effect to the surface of the preform. In addition, the amount of the hydrogen peroxide to be used can be reduced, and the remaining amount of the hydrogen peroxide on the surfaces of the preform 1 and the bottle 2 can be reduced.

As shown in Fig. 1(B), a nozzle 24 may be arranged below the preform 1 so that hydrogen peroxide mist K or gas similar to that supplied to the inner surface of the preform is blasted toward an outer surface of the preform 1.

Further, in the present invention, the preliminary sterilization treatment to the preform shown in Fig. 1(B) may be eliminated, and the bottle 2 is sterilized only in the main sterilization treatment which will be mentioned hereinafter.

As shown in Fig. 1(C), the preform 1 that has been preliminarily sterilized by the adhesion of the condensed film of the hydrogen peroxide is conveyed toward the return path of the endless chain 19, and heated by the infrared heaters 19a during the conveyance on the return path evenly to a temperature suitable for the blow molding till the time when the preform 1 is conveyed out of the heating furnace 50. This temperature is about in a range of 90 °C to 30 °C.

A heating temperature for heating the mouth portion 2a of the preform 1 is suppressed below 50 °C for preventing deformation which may be caused by the above reason.

The preform 1 subjected to the preliminary sterilization and heated to the temperature suitable for the blow molding is then molded to the bottle 2 as a blow-molded container as shown in Fig. 2(D).

The mold 4 as a shaping mold for the blow molding process is continuously travelled at the same travelling speed as that of the preform 1, the mold is clamped, and the mold is thereafter opened (released) after the blow-molding the preform 1 in the mold 4.

The preform 1 is heated to a temperature range suitable for the molding of the entire preform except the mouth portion 2a thereof in the heating process shown in (A) to (C) of Fig. 1, and while maintaining such heated state, the preform 1 is then disposed in the mold 4 together with the spindle 43 as shown in Fig. 2(D). The blow nozzle 5 is inserted into the preform 1 through a though hole 43a of the spindle 43.

During the travelling of the mold 4, for example, air for primary blowing process or secondary blowing process is subsequently blasted into the preform 1, and the preform 1 is thereby expanded in the cavity C of the mold 4 to a bottle 2 as a final product.

When the bottle 2 has been molded in the mold 4 as mentioned above, the mold is opened while travelling, and the completed product of the bottle 2 is taken out of the mold 4 as shown in Fig. 2(E).

The bottle 2 is then subjected to the main sterilization while continuously travelling after being molded as shown in Fig. 2(F). This main sterilization is performed by blasting the hydrogen peroxide mist M or gas G as a sterilizing agent from a sterilizing nozzle 6. The sterilizing nozzle 6 is positioned so as to oppose to the mouth portion 2a of the preform 1. The hydrogen peroxide mist M or gas G flows downward from the tip end of the sterilizing nozzle 6 into the bottle 2 through the bottle mouth portion 2a to thereby sterilize the inner surface of the bottle 2.

A tunnel 44 is formed at the continuously travelling path of the bottle 2, the hydrogen peroxide mist M or gas G blasted from the sterilizing nozzle 6 flows downward along the outer surface of the bottle 2, and is then stayed within the tunnel 44, thereby also effectively sterilizing the outer surface of the bottle 2.

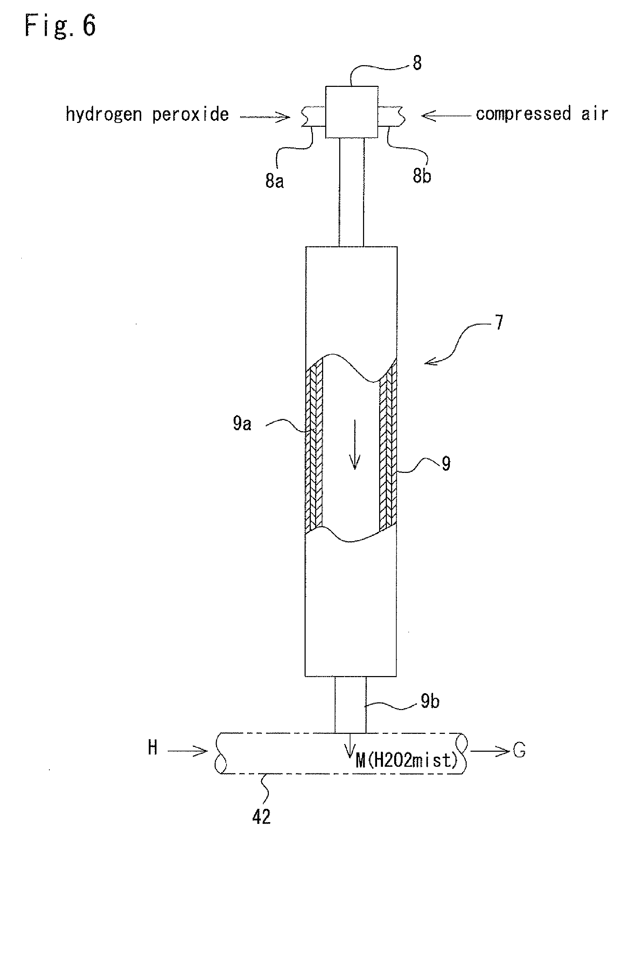

The hydrogen peroxide mist M or gas G may be generated by a mist generator 7, for example, shown in Fig. 6.

The mist generator 7 is provided with a hydrogen peroxide supply unit 8 as a two-fluid flow spray and an evaporator unit 9 for heating the hydrogen peroxide mist supplied from the hydrogen peroxide supply unit 8 to a temperature more than boiling point thereof but less than non-decomposition temperature and then evaporating the same.

The hydrogen peroxide supply unit 8 includes a hydrogen peroxide supply path 8a and a compressed air supply path 8b through which aqueous solution of the hydrogen peroxide and compressed air are introduced, respectively, and the aqueous solution of the hydrogen peroxide is then atomized into the evaporator unit 9. The evaporator unit 9 is composed of a pipe having inner and outer wall sections between which a heater 9a is embedded, and the atomized mist of the hydrogen peroxide blasted into this pipe is heated and then evaporated. The evaporated hydrogen peroxide gas G is discharged, as condensed mist, outside the evaporator unit 9 through a discharge nozzle 9b.

The mist M shown in Fig. 2(F) is this condensed mist. In a case when the gas G is utilized in place of such mist M, there may adopt the steps of, as shown in Fig 6 with two-dot and chain line, connecting a conduit 42 through which hot air-flow H flows is connected to the tip end of the discharge nozzle 9b, gasifying the condensed mist M discharged from the discharge nozzle 9b by the hot air-flow H, and flowing the gas G to the aforementioned sterilizing nozzle 6 by means of flexible hose or like.

The sterilizing nozzle 6 may be disposed at a predetermined position on the conveying path of the bottle 2 or may be moved in synchronism with the bottle 2. The number of the sterilizing nozzle 6 may be one or plural.

As shown in Fig. 2(F), the hydrogen peroxide mist M or gas G blasted from the sterilizing nozzle 6 contacts the inner and outer surfaces of the bottle 2, and in this state, the heat applied to the stage of the preform 1 remains on the bottle 2, which is hence maintained at the predetermined temperature by such remaining heat, so that the bottle 2 is effectively sterilized. This temperature is preferably in a range of 40 to 80 °C in the case of the preform being made of PET, and more preferably, 50 to 75 °C. Further, in the case of less than 40 °C, the sterilizing performance is extremely deteriorated and in the case of more than 80 °C, the bottle is contracted after the molding, thus being undesirable.

The hydrogen peroxide mist M or gas G used in this main sterilization is as follows.

### 1) Case of Using Hydrogen Peroxide:

In order to sterilize the bottle 2 to which only the main sterilization was conventionally performed, it was necessary to adhere the hydrogen peroxide, to the bottle 2, of the amount corresponding to 50 *µ* L/500 *µ*L bottle to 100 *µ*L/500*µ*L bottle. However, in the case when the preliminary heating followed with preheating of the preform 1 such as in the present invention, it becomes possible to achieve commercially allowable aseptic filling by adhering the hydrogen peroxide mist M of the amount corresponding to 10 *µ*L/500 *µ*L bottle to 50 *µ*L/500 *µ*L bottle blasting the hydrogen peroxide gas G having gas concentration of 1mg/L to 5mg/L.

### 2) Case of Using Hydrogen Peroxide Gas:

In order to sterilize the bottle 2 to which only the main sterilization was conventionally performed, it was necessary to blast the hydrogen peroxide gas G, to the bottle 2, having gas concentration of 5mg/l to 10mg/L. However, in the case when the preliminary heating followed with preheating of the preform 1 such as in the present invention, it becomes possible to achieve commercially allowable aseptic filling by blasting the hydrogen peroxide gas G having gas concentration of 1mg/L to 5mg/L.

After the blasting of the hydrogen peroxide mist M or gas G as mentioned above, the bottle 2 is continuously travelled, and as shown in Fig. 3(G1), the bottle 2 has an inverted attitude, and aseptic air N is blasted into the bottle 2 through the mouth portion 2a directed downward to thereby perform air rinsing treatment.

The air rinsing treatment is performed by blasting the aseptic air N into the bottle 2, It is preferred for the aseptic air N to be heated, and by blasting the heated aseptic air N into the bottle 2, the hydrogen peroxide supplied during the main sterilization treatment is activated to thereby improve the sterilizing effect to the bottle 2. It is desirable for the aseptic air N to be heated more than 70 °C in order to improve the sterilizing effect to the bottle 2.

Further, it may be preferred for a nozzle 45 to be inserted into the bottle 2 through the mouth portion 2a to a bottle transition region 2c. According to such insertion of the nozzle 45, the aseptic air N can be smoothly spread in the entire region inside the bottle 2. The transition region 2c may be defined as a region enlarged by about 70% of the maximum diameter of the bottle diameter from the lower end of the mouth portion 2a of the bottle 2.

Further, in the air rinsing treatment, exiting air present before the blasting of the aseptic air N is replaced with the blasting of the above-mentioned aseptic air N. As a result, surplus hydrogen peroxide contained in the existing air or foreign substance P existing inside the bottle 2 is discharged out of the bottle 2 through the mouth portion 2a. The foreign substance P discharged outside of the bottle 2 drops downward from the mouth portion 2a because the bottle 2 is inverted in attitude with the mouth portion 2a being now directed downward and is prevented from again entering into the bottle 2.

As shown in Fig. 3 (G2), the nozzle 45 may be arranged so as to oppose to the mouth portion 2a of the bottle 2 from the outside thereof without being inserted into the bottle 2 through the mouth portion 2a. As mentioned above, even in a case when the aseptic air N is blasted into the bottle 2 by arranging the nozzle 45 in a manner mentioned above, the hydrogen peroxide supplied in the bottle 2 can be activated as in the case shown in Fig. 3(G1), and the foreign substance P in the bottle 2 can be removed outside the bottle 2.

As described above, in the present invention, since the bottle 2 is subjected to the main sterilization treatment after the preliminary sterilization treatment in the stage of the preform 1, the amount of the hydrogen peroxide to be used in the main sterilization treatment can be reduced. Accordingly, the hot water rinsing treatment for rinsing away the hydrogen peroxide adhering to the bottle 2 by using hot water or like may be eliminated.

After the air rinsing treatment, as shown in Fig. 3 (H), the bottle 2 is turned into the positive erected attitude, and the bottle 2 is filled up with drink "a" through the filling nozzle 10, and thereafter, sealed with the cap 3 as a lid as shown in Fig. 3(I).

Thereafter, the bottles 2 formed as packaged bodies are collected and delivered to market.

A filling system for performing the above-mentioned filling method has a configuration, for example, as shown in Fig. 4.

As shown in Fig. 4, this filling system is provided with a preform supplying machine 11 for subsequently supplying preforms 1 (Fig. 1(A)), each having a bottomed cylindrical shape having a mouth portion 2a, a blow molding machine 12, and a filling machine 13 for filling the molded bottle 2 with a drink "a" and the bottle 2 is then sealed.

Between the preform supplying machine 11 and the filling machine 13, there are arranged a preform conveying unit for conveying the preform 1 on a first conveying path, a mold conveying unit for conveying the mold 4 (Fig. 2(D)) having a cavity C of a shape conforming with a shape of a product bottle 2 on a second conveying path connected to the first conveying path, and a bottle conveying unit for conveying the bottle 2 as a product molded by the mold 4 on a third conveying path connected to the second conveying path.

The first conveying path as the preform conveying unit, the second conveying path as the mold conveying unit and the third conveying path as the bottle conveying unit are communicated with each other, and grippers and other members, not shown, for conveying the preforms 1 and the bottles 2 while holding them are disposed on or along these conveying paths.

The preform conveying unit is equipped with preform conveyer 14 for subsequently feeding the preforms 1 at a predetermined interval on the first conveying path. The preform conveying unit is also equipped with a train of wheels 15, 16, 17, 18 which receive and then transfer the preforms 1 from the terminal end of the preform conveyer 14 and an endless chain 19 which receives the preforms 1 from the wheel 18 and then conveys them.

The endless chain 19 is arranged as the conveying path for conveying the preform 1 within the heating furnace 50 as mentioned above. The endless chain 19 is mounted with a number of spindles 43, shown in Fig. 1(A), at a predetermined interval between adjacent ones. Each spindle 43 travels together with the endless chain 19 while being rotated. The spindle 43 is inserted into the mouth portion 2a of the preform 1 conveyed on the endless chain side from the wheel 18 as shown in Fig. 1(A), and then, the preform is held by the spindle 43 in the positively erected attitude.

When the preform 1 is received by the spindle 43 through the belt conveyer 14 and the train of the wheels 15, 16, 17, and 18, the preform 1 travels, while rotating, along the inner wall section of the heating furnace 50. On the inner wall section of the heating furnace 50, the infrared heaters 19a are disposed in an entirely spread manner, and the preform 1 conveyed by the spindle 43 is heated by the infrared heaters 19a. The preform 1 is rotated together with the rotation of the spindle during the travelling within the heating furnace 50 and evenly heated by the infrared heaters 19a. The preform 1 is preheated for the preliminary sterilization within the heating furnace (Fig. 1(A)), during the travelling on the return path of the endless chain 19.

A hydrogen peroxide supply unit for blasting the hydrogen peroxide mist K or gas to the preform 1 is disposed in the vicinity of one 51a of the pulleys as a position being on an approximately middle portion of the conveying path of the preform 1 by the endless chain 19.

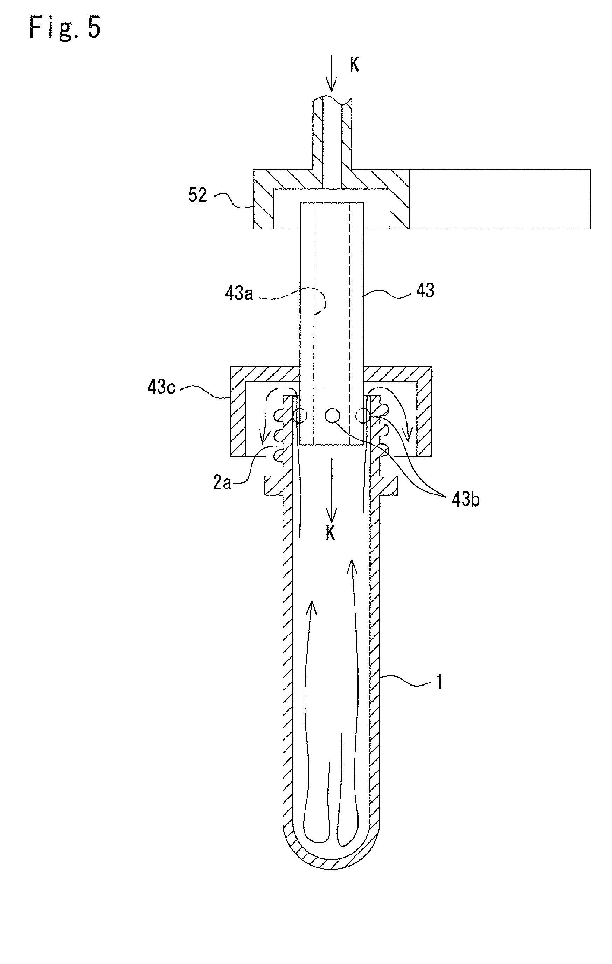

As shown in Fig. 5, the hydrogen peroxide supply unit is provided with a channel member 52 extending along the travelling path of the spindle 43 supporting the preform 1 so as to cover the upper end of the spindle 43. A blower 77 and a filter 78 are connected to the channel member 52 via a conduit as shown in Fig. 4, and another mist generator 79 similar to the mist generator 7 shown in Fig. 6 is connected to the conduit connecting the filter 78 and the channel member 52.

The hydrogen peroxide mist K or gas generated by the mist generator 79 is fed from the blower 77, enters the interior of the channel member 52 together with air stream sterilized by the filter 78, and then, flows into the preform 1 through the through hole 43a of the spindle 43. As shown in Fig. 5, a plurality of ball-shaped elastic materials made of rubber, for example, are embedded to the lower portion of the spindle 43, and according to the elastic deformation of these elastic materials 43b, the preform 1 is held by the spindle 43. The hydrogen peroxide mist K or gas flows out of the preform 1 through the gap between the inner peripheral surface of the mouth portion 2a of the preform 1 and the outer peripheral surface of the spindle 43.

According to the operation mentioned above, the condensed film of the hydrogen peroxide adheres to the inner surface of the preform 1 to thereby achieve the preliminary sterilization. As described hereinbefore, it is preferred that the adhering amount of 35 weight % conversion of the hydrogen peroxide condensed film is in a range of preferably of 0.001 *µ* L/cm² to 0.01 *µ* L/cm², and more preferably, 0.002 *µ* L/cm² to 0.008 *µ* L/cm², and the concentration of the hydrogen peroxide adhering to fungus (bacteria or like) body by the heat of the preheating to the preform 1 increases, and almost all the fungus adhering to the surface of the preform, except ones adhering to the mouth portion 2a of the preform 1, can be sterilized.

Further, as shown in Fig. 5, a guide member 43c is attached to the spindle 43 in an umbrella-like configuration so as to guide the hydrogen peroxide mist K or gas flowing outside the preform through the gap mentioned above toward the male thread formed to the mouth portion 2a of the preform 1. Owing to the guidance by the guide member 43c, the hydrogen peroxide mist K or gas is easily in contact with the male thread of the mouth portion 2a of the preform 1, and as a result, the outer surface of the mouth portion 2a of the preform 1 can be properly sterilized.

The preform 1 preliminarily sterilized enters the return path of the endless chain 19, and during the travelling, the preform 1 is further heated by the infrared heaters 19a, and the portions, except the mouth portion 2a, of the preform 1 are heated to a temperature of 90 °C to 130 °C that is suitable for the blow molding process.

The blow molding machine 12 is provided with plural sets of the molds 4 and the blow nozzles 5 (see Fig. 2(D)) for receiving the preforms 1 heated by the heating members 19a of the preform supply machine 11 and then molding the preforms 1 into the bottles 2.

The second conveying path of the mold conveying unit is provided within the blow molding machine 12, and the second conveying path is composed of a train of wheels 20, 21, 22, 17 and 23. Further, it is to be noted that the wheel 17 is commonly used between the train of these wheels 20, 21, 22, 17 and 23, and the train of the wheels 15, 16, 17 and 18 of the preform conveying unit.

The molds 4 and the blow nozzles 5 are arranged around the wheel 21 so as to turn around the wheel 21 together with the wheel 21 at a constant speed.

When a gripper, not shown, disposed for the wheel 20 receives the preform 1 together with the spindle 43 heated in the heating furnace of the preform supply machine 11 and then transfers the preform 1 to the mold 4 around the wheel 21, the mold 4, i.e., divided mold halves, is closed and grasps the preform in the state as shown in Fig. 2(D). The preform 1 inside the mold 4 is molded, while turning around the wheel 21 together with the mold 4 and the blowing nozzle 5, into a bottle 2 as a mold product by being subjected to the highly pressurized air-blow from the blowing nozzle 5. Since the preform 1 is heated to the predetermined temperature in the heating furnace 50 as shown in Fig. 1(C), the blow-molding process can be smoothly performed.

Further, as described above, since it is preferred that the adhering amount of 35 weight % conversion of the hydrogen peroxide condensed film is in a range of preferably of 0.001 *µ* L/cm² to 0.01 *µ* L/cm², and more preferably, 0.002 *µ* L/cm² to 0.008 *µ* L/cm², the condensed film of the hydrogen peroxide is evaporated and dispersed from the surface of the preform during the travelling on the return path of the endless chain 19. Thus, the blow-molding process can be properly performed without causing any whitening, spot, deformation and the like.

When the preform 1 is closely contacted inside the cavity C of the mold 4 and the bottle 2 is thereby formed, the mold 4 is opened when contacted to the wheel 22, and the bottle 2 and the spindle 43 are then released. Thereafter, the bottle 2 is transferred to a gripper, not shown, of the wheel 22 from the spindle 43.

Then, the spindle 43 from which the bottle 2 is released is returned to the conveyer 19 by way of the wheel 20, and subsequently holds and conveys another preform 1.

The bottle 2 delivered from the blow-molding machine 12 and reaching the wheel 22 is inspected by an inspection device 47 arranged around the outer periphery of the wheel 22 whether the molding defect is caused or not.

The inspection device 47 includes a bottle body inspection unit that judges a condition (good or defective, or right or wrong) of the body of the bottle 2, a support ring inspection unit that judges a condition of the support ring 2b of the bottle 2 (see Fig. 1(A)), a bottle neck ceiling inspection unit that judges a condition of the neck ceiling (top) portion of the bottle 2, a bottle bottom inspection unit that judges a condition of the bottom portion of the bottle 2, and a temperature inspection unit that detects the temperature of the bottle 2 to judges a condition of the bottle 2.

The bottle body inspection unit, the support ring inspection unit, the bottle neck ceiling inspection unit, and the temperature inspection unit are arranged along the outer periphery of the wheel 22.

The bottle body inspection unit, the support ring inspection unit, and the bottle neck ceiling inspection unit image various portions of the bottle 2 by means of lamps and cameras, not shown, the images being processed by an image processing device, and judges whether or not any defective in shape, injury, foreign substance, change in color or like exists.

The temperature inspection unit detects the temperature of the surface of the bottle 2 by a temperature sensor, not shown, and when the temperature does not reach the predetermined temperature, such bottle 2 is judged as defective one. That is, there is a possibility such that bottle 2 of which temperature does not reach the predetermined temperatures is not sufficiently sterilized in the sterilization treatment by the hydrogen peroxide. On the contrary, when the temperature of the bottle 2 reaches the predetermined temperature, it will be judged that the bottle 2 can be sufficiently sterilized by the sterilization performed thereafter by the hydrogen peroxide.

Further, the inspection device 47 may be arranged as occasion demands, and the bottle body inspection unit, the support ring inspection unit, the bottle neck ceiling inspection unit, and the temperature inspection unit may be provided selectively also as occasion demands.

When the inspected bottle 2 is judged as defective one, such bottle 2 is rejected by a rejecting device from the conveying path, and only the acceptable bottle 2 is conveyed to the wheel 23 through the wheels 22 and 17.

The nozzles 6 for the sterilization performing the main sterilization treatment to the bottles 2 are arranged around the outer periphery of the wheel 23 (see Fig. 2(F)). The sterilization of the bottle 2 is performed by blasting the hydrogen peroxide mist M or gas G from the sterilization nozzle 6 to the bottle 2 formed by the mold 4 and fungus or like remaining on the surface of the bottle 2 is sterilized. In this treatment stage, the heat due to the heating in the stage of the preform 1 remains, and by such heat, the sterilization effect by the hydrogen peroxide mist M or gas G can be enhanced.

As shown in Fig. 1(A) to (C), the preheating and the preliminary sterilization are performed to the preform 1 in the preform stage, and almost all the bacteria or like except one adhering to the mouth portion 2a can be sterilized by the preliminary sterilization. Accordingly, fungus or bacteria remaining in the preform stage in the blowing of the hydrogen peroxide mist M or gas G to the bottle 2 and little amount of fungus or bacteria mixed in the blow-molding process and conveying process can be sterilized in the main sterilizing treatment.

In the sterilization treatment by the hydrogen peroxide mist M or gas G, since the preliminary sterilization is performed in the stage of the preform 1, the amount of the hydrogen peroxide to be used can be reduced.

The filling machine 13 is provided inside thereof with the third conveying path of the bottle conveying unit. The third conveying path is provided with a train of wheels 27, 34, 35, 36, 37, and 38.

The air rinsing nozzle 45 (see Fig. 3(G1)) is arranged around the outer periphery of the wheel 27 in an attitude directed upward, and a gripper gripping the bottle 2 is arranged to a portion corresponding to the nozzle 45 so as to travel around the wheel 27 in a vertically inverted attitude. As an inverting mechanism therefor is known one, detailed explanation thereof is omitted herein.

The bottle into which the hydrogen peroxide mist M or gas G is blasted is grasped by the gripper of the wheel 27, and travels in an inverted attitude. When the bottle reaches a position directly above the nozzle 45, the heated aseptic air N for air rinsing treatment is blasted into the bottle 2, directed downward, through the mouth portion 2a thereof

(Fig. 3(G1)). According to such operation, the hydrogen peroxide existing inside the bottle 2 is activated, thus enhancing the sterilization effect. In addition, as a result of smooth replacement of the existing air in the bottle with the above-mentioned aseptic air N, the surplus hydrogen peroxide and foreign substance P within the bottle are discharged outside the bottle 2 through the mouth portion 2a directed downward.

Although the aseptic air N is supplied inside the bottle 2 by inserting the nozzle 45 into the bottle 2 as shown in Fig. 3 (G1), the aseptic air N may be supplied from the outside of the bottle 2 as shown in Fig. 3(G2).

Around a next wheel 35, a filler 35 is provided for filling the drink "a" into the bottle 2 now in the aseptic state, and around a wheel 37, a capper 40 is provided for applying the cap 3 to the bottle 2 filled with the drink "a" to seal the same.

Further, since known filler and capper are usable for the filler 39 and the capper 40, the explanation thereof is omitted herein.

The filling system is surrounded by a chamber 41, which is sectioned into an aseptic zone and a gray zone. The preform supply machine 11 and the blow-molding machine 12 are arranged in the gray zone and the filling machine 13 is arranged in the aseptic zone, respectively.

Aseptic air by the sterilization in the HEPA is always continuously blasted into the gray zone, thereby transferring the bottle 2 sterilized in the molding process into the aseptic zone without being secondarily contaminated by bacteria or like.

Next, the operation of the filling system will be explained with reference to Figs. 1 to 6.

First, the preform 1 is conveyed into the heating furnace 50 via the preform conveyer 14 and the train of the wheels 15, 16, 17, 18.

When the preform 1 enters the heating furnace 50, the preform 1 is preliminarily heated for the preliminary sterilization on the way of the return path of the endless chain 19 (Fig. 1(A)).

The hydrogen peroxide mist K or gas is blasted into the preheated preform 1, as shown in Fig. 1(B) and Fig. 5, at the terminal end of the outward path of the endless chain 19. According to such blasting, thin film of the condensed hydrogen peroxide is formed on the inner surface of the preform. Since the preform 1 is preheated to the temperature suitable for preliminary sterilization, the hydrogen peroxide adhering to the preform is activated, thus enhancing the sterilization effect.

The preform 1 is further heated in the heating furnace 50, and the entire structure of the preform 1, except the mouth portion 2a, is evenly heated to a temperature range suitable for the blow molding process.

The preform 1 preheated in the heating furnace 50, preliminarily sterilized, and then heated to the molding temperature is held in the mold 4 as shown in Fig. 2(D) during the passing around the outer periphery of the wheel 21, and swelled into the cavity C of the mold by the blowing of the highly-pressurized air from the blow nozzle 5 to thereby form a completed product.

The molded bottle 2 is taken out of the mold 4 by the gripper of the wheel 22 after opening the mold 4, and after the inspection of the inspection device 47 as to whether defective one exists or not in the molding process, the main sterilization treatment is performed as shown in Fig. 2(F) during the passing around the outer periphery of the wheel 23, and the hydrogen peroxide gas G or mist K is blasted from the sterilizing nozzle 6. The hydrogen peroxide gas G or mist M flows into the bottle 2 through the mouth portion 2a thereof and flows downward in contact with the outer surface of the bottle 2.

Since the heat applied in the heating furnace 50 remains on the surface of the bottle 2, the bottle 2 can be effectively sterilized by the hydrogen peroxide gas G or mist M blasted through the sterilizing nozzle 6. According to such sterilization, fungus or bacteria alive on the surface of the preform 1 can be sterilized and removed.

The thus sterilized bottle 2 is delivered toward the downstream-side wheel 27 from the wheel 23, and is rinsed by air around the wheel 27. That is, the bottle 2 is inverted during the passing around the wheel 27 as shown in Fig. 3(G1) or (G2), and the heated aseptic air N is blasted into the bottle 2 from the nozzle 45 that is inserted into the bottle 2 through the mouth portion 2a thereof or opposing to the mouth portion 2a on the outside the bottle 2.

The hydrogen peroxide existing inside the bottle 2 is activated by the heated aseptic air N to thereby improve the sterilizing function. Further, at a time when the existing air inside the bottle 2 is replaced with the aseptic air N at a high speed, in a case where any foreign substance P is present in the bottle 2, the foreign substance P is discharged outside the bottle 2 through the mouth portion 2a directed downward as shown in Fig. 3(G1) or (G2).

Further, the flow rate of the aseptic air N for the air-rinsing treatment blasted into the bottle 2 from the nozzle 45 is preferably in a range of 100L/min. to 500L/min., more preferably, 150L/min. to 300L/min., with respect to the bottle of inner volume of 50mL to 5000mL or 200mL to 2000mL, and the aseptic air N is discharged preferably for 3 to 20 sec., more preferably, 3 to 10 sec.

Furthermore, the heating temperature to the aseptic air N, i.e., temperature at the time when the aseptic air is discharged from the nozzle 45 is preferably 50 °C to 150 °C, more preferably, 75 °C to 120 °C.

Thereafter, the bottle 2 travels within the filling machine 13 while being transferred to the train of the wheels 34, 35, 36, 37 and 38.

Within the filling machine 13, the bottle 2 is filled up with the sterilized drink "a" through the filling nozzle 10 of the filler 39 as shown in Fig. 3(H). The bottle 2 filled up with the drink "a" is applied and sealed with the cap 3 by the capper 40 (Fig. 3(I)), and then discharged through the outlet of the chamber 41.

As mentioned hereinbefore, since the filler 39 and the capper 40 are known ones, explanations of the method of filling the bottle 2 with the drink and the method of sealing the bottle 2 are hence eliminated herein.

### <Embodiment 2>

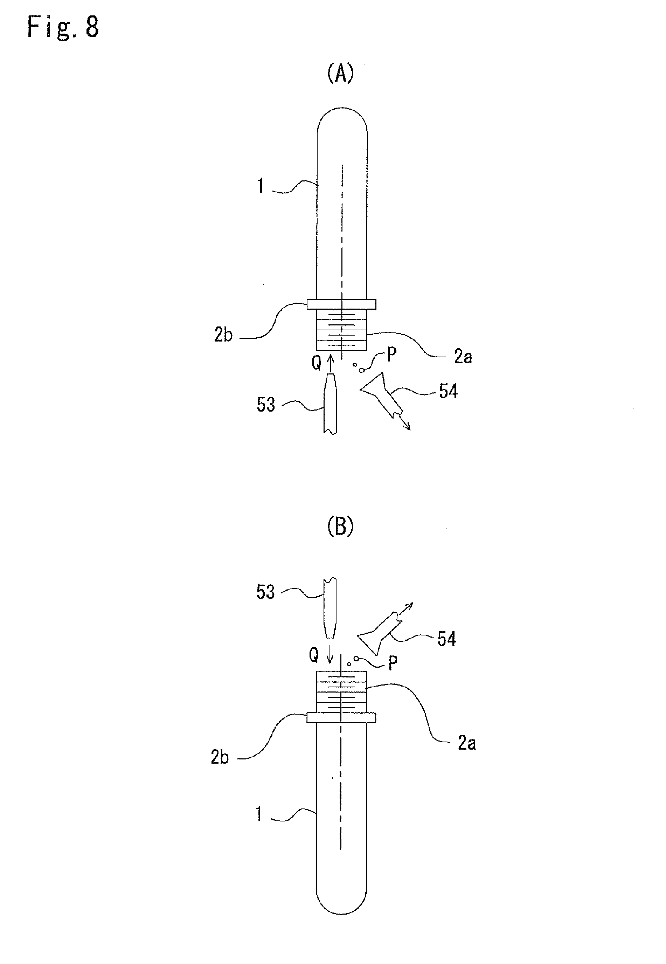

Although, in the embodiment 1 mentioned above, the preform 1 is heated to a temperature suitable for the molding process, as shown in Fig. 1(C) during the conveyance of the preform 1, in the present embodiment 2, an air-rinsing treatment is performed by blasting air Q from an air nozzle 53 into the preform 1, as shown in Fig. 8(A), before heating the preform 1 to the temperature suitable for the molding treatment as shown in Fig. 1(C), preferably, before the preliminary heating at the outside of the heating furnace 50 as shown in Fig. 1(A), thereby removing the foreign substance P from the inside of the preform 1.

As shown in Fig. 8(A), at an instance when the air Q is blasted from the air nozzle 53, the preform is inverted such that the mouth portion 2a thereof is directed downward. Further, since the preform 1 has a test-tube shape having a smooth vertical inner surface, the foreign substance P present inside the preform 1 falls downward smoothly outside the preform by the gravity thereof.

Although atmospheric air may be used as such air Q in place of the aseptic air, it is preferred to use compressed air, as the air Q, of an amount of 1µm to 0.22 µm, which was filtrated by an air filter for sterilization.

Moreover, the air nozzle 53 may be arranged on en extension line of the center line of the preform 1 or may be arranged on a deflected portion slightly apart from the center line of the preform 1. According to such arrangement, the foreign substance P is swiveled by the air Q discharged from the air nozzle 53 to be thereby accurately released outside the preform 1.

The preform 1 from which the foreign substance P is removed as mentioned above is thereafter molded into a container such as bottle 2, and the foreign substance P is more surely removed from the interior of the bottle 2 by blasting the aseptic air N through the nozzle 45.

At a time when the air is blasted into the preform 1, the posture changing (change of attitude) of the bottle 2 from the erected attitude to the inverted attitude or vice versa can be easily done by arranging a mechanism for turning the gripper holding the preform 1 in a vertical plane, and since a known mechanism can be used as such mechanism, explanation thereof may be eliminated herein.

Further, as shown in Fig. 8(B) as an alternation, it may be possible to convey the preform in an erected attitude and to blast the air Q through the air nozzle 53 into the preform from the mouth portion 2a directed upward.

In addition, in either case shown in Fig. 8(A) or (B), it may be desired that the foreign substance P once discharged out of the preform 1 by blasting the air Q is captured and collected by the air nozzle 54 of the suction device so as not to again enter the preform 1.

Furthermore, it may be possible to arrange the air nozzle 53 and the suction nozzle 54 to desired positions of the preform conveyer 14, and the wheels 15, 16, 17 and 18 constituting the conveying path for the preform 1.

### Example 1

An example of an air-rinsing with respect to the Exemplary Embodiment 1 will be described.

Kinds of the foreign substance P shown in Fig. 3(G1) or (G2) and supply amount of the aseptic air N into the bottle are shown in the following Table 1.

**[Table 1]**

| | foreign substance sample size | size | initial injection amount | kind of bottle | nozzle diameter | nozzle insertion distance | rinsing time (sec) | air-flow amount (L/min) | calculation method of foreign substance removal |
|---|---|---|---|---|---|---|---|---|---|
| 1 | resin yarn A | *φ* 0.148mm × 5mm | 64threads | 500mL or 2L | inner diameter 6mm *φ* outer diameter 8mm *φ* | 30mm(from bottle ceiling surface) | 3 | 150 or 200 | (removing amount/initial injection amount) × 100 |
| 2 | resin yarn B | *φ* 0.185mm × 5mm | 64threads | | | | | | |
| 3 | rubber packing | thickness 0.5mm × 2mm | 10pieces | | | | | | |
| 4 | PET film | thickness 0.1 mm × 5mm square | 10pieces | | | | | | |
| 5 | resin yarn C | *φ* 0.55mm × 5mm | 64threads | | | | | | |

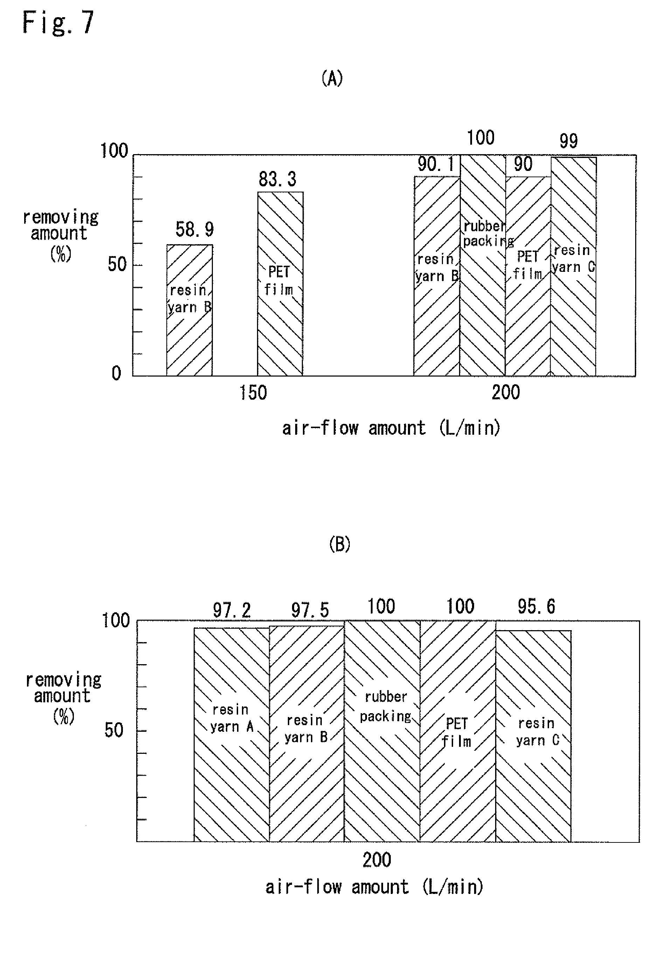

First, five bottles each having volume of 2L (litter) were prepared. Sample of foreign substance 2 (resin yarn B) and sample of foreign substance 4 (PET film) were injected, respectively, into the bottles. Then, the bottles were inverted in order and air of flow rate of 150L/min. was blasted into the bottle through the mouth portion thereof for 3 sec. As the blasted air, aseptic air heated to a temperature more than 70 °C was used. As a result, the foreign substance removing ratio was, as shown in left side on Fig. 7(A), 58.9% with respect to the foreign substance sample 2 (resin yarn B) and 83.3% with respect to the foreign substance sample 4 (PET film).

Next, bottles each having volume of 2L, into which sample of foreign substance 2 (resin yarn B), sample of foreign substance 3 (rubber packing), sample of foreign substance 4 (PET film) and sample of foreign substance 5 (resin yarn C) were injected respectively, were inverted in order, and then, heated aseptic air of flow rate of 200L/min. was blasted into the bottle through the mouth portion thereof for 3 sec. In such case, the foreign substance removing ratio was, as shown in right side on Fig. 7(A), 90.1% with respect to the foreign substance sample 2 (resin yarn B), 100% with respect to the foreign substance sample 3 (rubber packing), 90% with respect to the foreign substance sample 4 (PET film), and 99% with respect to the foreign substance sample 5 (resin yarn C).

Furthermore, bottles each having volume of 500mL, into which sample of foreign substance 1 (resin yarn A), sample of foreign substance 2 (resin yarn B), sample of foreign substance 3 (rubber packing), sample of foreign substance 4 (PET film) and sample of foreign substance 5 (resin yarn C) were injected respectively, were inverted in order, and then, heated aseptic air of flow rate of 200L/min. was blasted into the bottle through the mouth portion thereof for 3 sec. In such case, the foreign substance removing ratio was, as shown in right side on Fig. 7(B), 97.2 with respect to the foreign substance sample 1 (resin yarn A), 97.5% with respect to the foreign substance sample 2 (resin yarn B), 100% with respect to the foreign substance sample 3 (rubber packing), 100% with respect to the foreign substance sample 4 (PET film), and 95.6% with respect to the foreign substance sample 5 (resin yarn C).

With the above foreign substance removing rates were calculated by reducing the number of the foreign substances discharged out of each bottle from the number of the foreign substances initially injected into the bottle.

From the results mentioned above, it was found that the foreign substance removing rate exceeds 90% with respect to the bottle having volume of 2L, by blasting the air of 200L/min. into the bottle for 3 sec., and the foreign substance removing rate exceeds 90% with respect to the bottle having volume of 500mL, by blasting the air of 200L/min into the bottle for 3 sec.

In view of the above results, it will be considered that the sterilizing method of the present embodiment provides the foreign substance removing rate over 90% with respect to the foreign substances of the kinds and sizes mentioned above, the hydrogen peroxide is decomposed and removed while being activated by using the heated aseptic air, and is the most economical method for manufacturing bottled drink.

It is further to be noted that, with respect to the bottles having volumes of 500mL and 2L, in a case where the hydrogen peroxide was adhered as mentioned hereinbefore, and thereafter, inverted, and then, the heated aseptic air of 200L/min. was blasted for 3 sec., the obtained sterilized result was more than 610g with respect to B.subtiLis spore.

Moreover, in a result in which distilled water filled the abovementioned bottles after the removal of the foreign substance, and thereafter, the hydrogen peroxide concentrations in the distilled water were less than 0.5ppm. This value satisfies the standard determined by the U.S. FDA.

### Example 2

An example of an air-rinsing with respect to the Exemplary Embodiment 2 will be described hereunder.

**[Table 2]**

| function | air-rising time | | |
|---|---|---|---|
| | 0 - 3sec. | 3 - 10sec | more than 10sec |
| ① bottle contractile property | ⊚ | ○ | × |
| ② remaining H₂O₂ reducing effect | × | ○ | ⊚ |
| ③ sterilizing effect | × | ○ | ⊚ |
| ④ foreign substance removing performance | Δ | ○ | ⊚ |
| ⑤ cost | ⊚ | ○ | × |
| **total evaluation** | **×** | **○** | **×** |

Standard for Criteria is as follows.
(A) Contractile Property (Bottle Contracting Amount) (Refer to Fig. 9(A)).
   less than 0.2%: ⊚
   0.2 to 1%: ○
   more than 1%: X
(B) Remaining Hydrogen Peroxide Reduction Effect
   (Amount of Hydrogen Peroxide Remaining in Bottle) (Refer to Fig. 9(B))
   more than 0.5ppm: X
   0.1-0.5ppm: ○
   less than 0.1ppm: ⊚
(C) Sterilization Effect (Sterilization Effect to B.subtiLis Spore)
   (Refer to Fig. 9(C))
   less than 6LRV (Log Reduction Value): X
   6 to 7 LRV: ○
   more than 7LRV: ⊚
(D) Foreign Substance Removing Performance (Resin Yarn Removing Rate) (Refer to Fig. 9(D))
   less than 90%: Δ
   90 to 95%: ○
   more than 95%: ⊚
(E) Cost (Total Cost: Order of Investment Cost + Running Cost + Maintenance Cost) (Refer to Fig. 9(E)
   low: ⊚
   middle: ○
   high: X

It is further to be noted that the present invention is not limited to the embodiments described above and may be carried out by many other various embodiments or modes. For example, a container to which the present invention is applied is not limited to a PET bottle, and many other containers made of resin may be usable. Moreover, the molding process may be performed without being limited to the injection blow molding, and various other blow-molding process such as direct blow process may be adopted. Furthermore, the conveying means for conveying the preform and the container is not limited to the wheel conveying train illustrated in Fig. 4, and various conveying means capable of sequentially conveying the formed containers at a predetermined speed, for example, belt conveyer, bucket conveyer, pneumatic (air) conveyer and the like may be also utilized.

### Reference Numerals

- 1: preform
- 2: bottle
- 3: cap
- 4: mold
- 6: main sterilization nozzle
- 19: endless chain
- 39: filler
- 40: capper
- 43a: through hole of spindle
- 45: nozzle for air-rinse
- 50: heating furnace
- "a": drink
- G: hydrogen peroxide gas
- M: hydrogen peroxide mist
- K: hydrogen peroxide mist
- N: aseptic air
- P: foreign substance

## Claims

1. A drink filling method comprising the steps of:
blasting hydrogen peroxide mist or gas into a container in an erected attitude through a mouth portion thereof;
activating the hydrogen peroxide in the container in an inverted attitude by blasting heated aseptic air through the mouth portion thereof directed downward, and simultaneously, removing foreign substance from the container by replacing air in the container with the aseptic air;
filling the container in the erected attitude with drink through the mouth portion directed upward; and
closing the mouth portion of the container by applying a lid to the mouth portion of the container.

2. The drink filling method according to claim 1, wherein, while continuously traveling the preforms, each of the preforms is heated to a temperature suitable for molding, the preform is blow-molded into the container, and steps from the hydrogen peroxide mist or gas blasting step to the container mouth portion closing step are subsequently performed with respect to the container having remaining heat at the time of above-mentioned heating step.

3. The drink filling method according to claim 2, wherein, while continuously traveling the preforms, the hydrogen peroxide mist or gas is contacted to the preforms and steps form the preform heating step in which the preform is heated to the molding temperature to the container mouth portion closing step are subsequently performed.

4. The drink filling method according to claim 1, wherein the aseptic air is blasted into the container by a flow rate of 100L/min. to 500L/min. for 3 to 20 sec.

5. The drink filling method according to claim 2 or 3, wherein air is blasted into the preform before heating the preform to the molding temperature to thereby remove foreign substance from the preform.

6. A drink filling system, in which there is provided a conveying path for continuously travelling a preform and a container during processes in which a preform is formed to a container, a drink fills the container, the container is sealed by a lid, and for traveling the container in a temporarily inverted attitude, the drink filling system comprising: a heating furnace for heating the preform to a temperature suitable for molding; a mold for blow-molding the heated preform into the mold; a sterilizing nozzle for blasting the hydrogen peroxide mist or gas into the blow-molded container through the mouth portion thereof; an air-rinsing nozzle for activating the hydrogen peroxide inside the container by blasting the heated aseptic air through the mouth portion directed downward of the container traveling in the inverted attitude and substantially reducing the air inside the container with the aseptic air to thereby remove the foreign substance from the container; a drink filling nozzle for filling the container with the drink; and a capper for closing the mouth portion of the container which is filled up with the drink, which are arranged along the conveying path.

7. The drink filling system according to claim 6, wherein an air nozzle for removing the foreign substance from the inside of the preform by blasting air into the preform before heating the preform to the molding temperature is provided for the conveying path.

8. The drink filling system according to claim 7, wherein a suction nozzle for sucking the foreign substance discharged from the inside of the preform is provided for the conveying path.
